(19) Europäisches Patentamt

European Patent Office

Office européen des brevets

(11) **EP 1 521 079 A1**

(12) **DEMANDE DE BREVET EUROPEEN**

(43) Date de publication:
**06.04.2005 Bulletin 2005/14**

(51) Int Cl.7: **G01N 30/46**, G01N 30/86

(21) Numéro de dépôt: **04300637.8**

(22) Date de dépôt: **29.09.2004**

| | |
|---|---|
| (84) Etats contractants désignés:<br>**AT BE BG CH CY CZ DE DK EE ES FI FR GB GR HU IE IT LI LU MC NL PL PT RO SE SI SK TR**<br>Etats d'extension désignés:<br>**AL HR LT LV MK**<br><br>(30) Priorité: **03.10.2003 FR 0311636**<br><br>(71) Demandeur: **Actaris Gaszählerbau GmbH 76185 Karlsruhe (DE)** | (72) Inventeur: **Aprahamian, Edouard 69280, MARCY L'ETOILE (FR)**<br><br>(74) Mandataire: **Feray, Valérie et al Feray Lenne Conseil 39/41, avenue Aristide Briand 92160 Antony (FR)** |

(54) **Procédé d'identification de composants d'un gaz naturel par chromatographie en phase gazeuse**

(57) La présente invention concerne un procédé d'identification par chromatographie en phase gazeuse d'au moins deux composants $C_d$ et $C_k$ appartenant à un Gaz Naturel, lesdits deux composants $C_d$ et $C_k$ étant retenus dans une première colonne de chromatographie, ledit procédé comportant les étapes suivantes :

- injection d'un premier échantillon dudit Gaz Naturel dans ladite première colonne de chromatographie,
- séparation desdits composants $C_d$ et $C_k$ à l'intérieur de ladite première colonne,
- détection desdits composants $C_d$ et $C_k$ sous la forme d'un premier chromatogramme représentant au moins deux pics successifs $P_d$ et $P_k$,

- détermination du temps mort Tm à partir dudit premier chromatogramme,
- détermination de la rétention relative $\alpha_{dk}$ entre lesdits pics $P_d$ et $P_k$ définie par la relation : $\alpha_{dk} = \frac{Tr(d)-Tm}{Tr(k)-Tm}$, Tr(d) et Tr(k) désignant respectivement les temps de rétention associés aux dits pics $P_d$ et $P_k$,
- identification des composants $C_d$ et $C_k$ en déterminant l'appartenance de ladite rétention relative $\alpha_{dk}$ à un intervalle connu de rétentions relatives.

Printed by Jouve, 75001 PARIS (FR)

**Description**

La présente invention concerne un procédé d'identification par chromatographie en phase gazeuse d'une pluralité de composants appartenant à un échantillon de Gaz Naturel. Le procédé selon l'invention est plus particulièrement adapté à être utilisé dans un compteur d'énergie de Gaz Naturel.

De manière générale, le Gaz Naturel est un mélange gazeux d'hydrocarbures saturés, où domine le méthane, et de composants oxygénés, azotés ou sulfurés. La mesure de la composition du Gaz Naturel en différents points critiques au cours de la production et de la distribution devient de plus en plus nécessaire. Il se peut en effet que la composition du Gaz Naturel varie de façon notable entraînant une variation du pouvoir calorifique du gaz. L'analyse de la composition du gaz permet de calculer le pouvoir calorifique réel du Gaz Naturel en fonction de sa composition.

Afin de procéder à l'analyse de Gaz Naturel, on utilise la chromatographie en phase gazeuse.

Cette technique permet de séparer des mélanges gazeux complexes par suite continue d'équilibres s'établissant entre une phase mobile gazeuse et une phase stationnaire appropriée.

De manière connue, un chromatographe comporte:

- un dispositif d'injection,
- une enceinte thermostatée,
- une colonne de chromatographie,
- un détecteur.

Le gaz à analyser est injecté dans la colonne de chromatographie tandis que le gaz vecteur balaye cette même colonne en continu ; le gaz vecteur est également appelé phase mobile.

La colonne de chromatographie contient également un produit permettant la séparation des différents composants du gaz à analyser : ce produit est appelé phase stationnaire. La colonne est maintenue à température constante dans une enceinte thermostatée.

Le gaz à analyser traverse la phase stationnaire poussé par la phase mobile. Les différents composants du gaz à analyser n'ayant pas les mêmes interactions avec chacune des phases, sont ainsi séparés par différence de vitesse de migration le long de la colonne ; les composants sont retenus de manière différente dans la colonne.

Le type de séparation dépend de la colonne utilisée. Ainsi, pour un même Gaz Naturel, on peut utiliser une première colonne, notée CB par la suite, pour séparer une première série d'hydrocarbures lourds tels que le propane $C_3H_8$, l'iso-butane $iC_4H_{10}$, le normal-butane $nC_4H_{10}$, le néo-pentane $néoC_5H_{12}$, l'Iso-pentane $iC_5H_{12}$, le normal-pentane $nC_5H_{12}$, les hexanes et les composants supérieurs en $C_7$, $C_8$ et $C_9$ notés par la suite $C_{6+}$ et une deuxième colonne, notée CA par la suite, permettant de séparer une deuxième série de composants plus légers tels que l'azote $N_2$, le méthane $CH_4$, le dioxyde de carbone $CO_2$, l'éthane $C_2H_6$ et le propane $C_3H_8$. Notons que la deuxième colonne CA permet également de mesurer l'eau $H_2O$.

La colonne CB est une colonne de chromatographie de partage qui se comporte comme une colonne à distiller et qui sépare les composants suivant leurs points d'ébullition. Le composant à mesurer est en partie dissout dans la phase stationnaire qui est un film de liquide déposé sur la paroi interne de la colonne. Le rapport de distribution du composant entre la phase stationnaire et la phase mobile est appelé coefficient de partage.

La colonne CA est une colonne de chromatographie d'adsorption; dans ce cas, la phase stationnaire est un solide déposé sur la paroi interne de la colonne. La surface de cette phase stationnaire se trouve recouverte de molécules de la phase mobile. L'adsorption des molécules du composant se trouve réalisée par la désorption d'un certain nombre de molécules de la phase mobile et la fixation des molécules du composant est réalisée par le jeu des forces d'interaction diverses entre les groupements fonctionnels du composant et les atomes de la phase stationnaire.

Il faut ensuite détecter les différents composants lorsqu'ils sortent de la colonne ; cette détection est réalisée par un détecteur à conductibilité thermique : ce type de détecteur comporte un pont de Wheatstone dont les branches sont des résistances thermosensibles. La détection des composants se fait par la mesure du déséquilibre électrique du pont de Wheatstone.

Le déséquilibre du pont donne lieu à un signal se présentant sous la forme d'un pic ayant une allure sensiblement gaussienne.

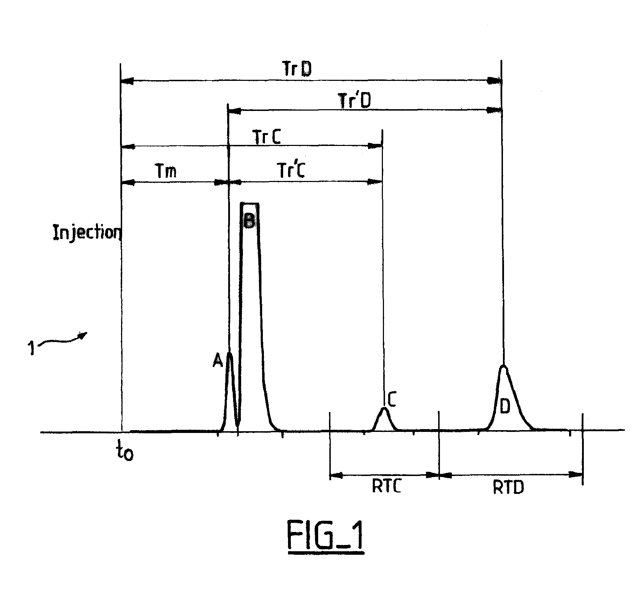

Le résultat de la détection est ensuite représenté sur un chromatogramme tel que le chromatogramme 1 représenté en figure 1.

Le chromatogramme 1 comprend un ensemble de quatre pics A, B, C et D représentés en fonction du temps.

Le temps t0 correspond au temps de départ de l'injection de l'échantillon de gaz à analyser.

Chacun des pics correspond à un composant du Gaz Naturel à analyser. Il faut alors identifier le composant correspondant à chacun des pics sachant que ces composants sortent toujours dans le même ordre.

Une solution connue à ce problème consiste à utiliser des fenêtres d'identification pour chacun des pics.

Chaque pic définit un temps de rétention Tr qui est le temps écoulé depuis le moment de l'injection t0 jusqu'au temps au sommet du pic.

Les temps de rétention TrC et TrD sont ainsi représentés sur la figure 1.

Le composant A est considéré ici comme un pic non retenu dans la colonne de mesure.

Le temps de rétention du composant A correspond ainsi au temps mort de la colonne, noté Tm, c'est à dire au temps de passage dans la colonne d'un composant non retenu.

Des fenêtres temporelles d'identification RT définissent des intervalles de temps de rétention Tr déterminés expérimentalement et correspondant à un composant identifiable. Les fenêtres RTC et RTD respectives des composants C et D sont représentées en figure 1. Le temps de rétention TrC appartenant à la fenêtre RTC permet d'identifier le composant C correspondant. De façon identique, le temps de rétention TrD appartenant à la fenêtre RTD permet d'identifier le composant D correspondant.

Ainsi, la détermination d'un temps de rétention Tr et son appartenance à une certaine fenêtre d'identification permettent d'identifier un composant.

Toutefois, la mise en oeuvre d'une telle solution présente certaines difficultés.

Ainsi, les temps de rétention sont fortement dépendants de la température de la colonne ainsi que du débit du gaz vecteur qui la traverse.

En conséquence, la moindre variation de température ou de débit de gaz vecteur peut entraîner la sortie du temps de rétention de la fenêtre d'identification et l'impossibilité d'identifier le composant correspondant ; les résultats de l'analyse sont alors totalement erronés. A titre indicatif, une variation de température supérieure à 1°C ou une variation de débit de gaz vecteur supérieure à 5% peuvent entièrement fausser l'analyse.

La présente invention vise à fournir un procédé d'identification par chromatographie en phase gazeuse d'une pluralité de composants appartenant à un échantillon de Gaz Naturel sans se préoccuper des éventuelles dérives des temps de rétention dues à une variation de température de la colonne ou à une variation du débit de gaz vecteur.

La présente invention propose à cet effet un procédé d'identification par chromatographie en phase gazeuse d'au moins deux composants $C_d$ et $C_k$ appartenant à un Gaz Naturel, lesdits deux composants $C_d$ et $C_k$ étant retenus dans une première colonne de chromatographie, ledit procédé comportant les étapes suivantes :

- injection d'un premier échantillon dudit Gaz Naturel dans ladite première colonne de chromatographie,
- séparation desdits composants $C_d$ et $C_k$ à l'intérieur de ladite première colonne,
- détection desdits composants $C_d$ et $C_k$ sous la forme d'un premier chromatogramme représentant au moins deux pics successifs $P_d$ et $P_k$,
- caractérisé en ce que ledit procédé d'identification comporte les étapes suivantes :

- détermination du temps mort Tm à partir dudit premier chromatogramme,
- détermination de la rétention relative $\alpha_{dk}$ entre lesdits pics $P_d$ et $P_k$ définie par la relation :

$$\alpha_{dk} = \frac{Tr(d)\text{-}Tm}{Tr(k)\text{ - }Tm},$$

Tr(d) et Tr(k) désignant respectivement les temps de rétention associés aux dits pics $P_d$ et $P_k$,

- identification des composants $C_d$ et $C_k$ en déterminant l'appartenance de ladite rétention relative $\alpha_{dk}$ à un intervalle connu de rétentions relatives.

Grâce à l'invention, on utilise un intervalle (ou fenêtre) de rétentions relatives et on identifie les composants $C_d$ et $C_k$ du Gaz Naturel par l'appartenance de leur rétention relative à ladite fenêtre. La rétention relative varie en effet très peu avec la température de la colonne et le débit du gaz vecteur, notamment pour la colonne de chromatographie, notée CB, permettant de séparer les hydrocarbures tels que le propane $C_3H_8$, l'iso-butane $iC_4H_{10}$, le normal-butane $nC_4H_{10}$, le néo-pentane $néoC_5H_{12}$, l'iso-pentane $iC_5H_{12}$ et le normal-pentane $nC_5H_{12}$ du Gaz Naturel. Dès lors, une variation de température de la colonne ou de débit du gaz vecteur ne risque pas d'entraîner une sortie, hors de la fenêtre de rétention, de la rétention relative mesurée correspondante, comme cela peut être le cas pour des fenêtres d'identification utilisant les temps de rétention. A titre indicatif, une variation de température de la colonne de +/-10°C ou une variation de débit de gaz vecteur de +/-20% ne perturbe pas l'identification des pics d'hydrocarbures séparés correspondant aux composants $C_3H_8$, $iC_4H_{10}$, $nC_4H_{10}$, $iC_5H_{12}$ et $nC_5H_{12}$ du Gaz Naturel. On peut ainsi déterminer, de manière très stable, lesdites fenêtres de rétention pour chaque couple de composants $C_d$ et $C_k$ et identifier ensuite avec certitude, les pics en fonction de l'appartenance de leur rétention relative aux différentes fenêtres.

Avantageusement, ledit premier chromatogramme comporte un premier pic dit composite, correspondant à un composant non retenu, ledit temps mort Tm étant assimilé au temps de démarrage dudit pic composite.

La détermination de la rétention relative passe en effet par la détermination du temps mort Tm. Ainsi, dans le cas de la colonne CB, le chromatogramme comporte un premier massif incluant des composants non retenus tels que l'hélium, l'hydrogène, l'argon, l'oxygène, l'azote, le monoxyde de Carbone, le méthane et le dioxyde de carbone. Le temps de démarrage de ce pic, dit composite, peut être assimilé au temps mort Tm de la colonne. Dans la pratique, le temps mort utilisé correspond à une première déviation de la ligne de base (obtenue par la dérivée seconde du signal), déviation qui précède immédiatement une saturation du signal ou un niveau de signal supérieur à une valeur prédéterminée.

De manière particulièrement avantageuse, ledit échantillon de Gaz Naturel comporte n composants $C_i$ retenus dans ladite première colonne de chromatographie, n étant un entier strictement supérieur à 1 et i variant de 0 à n-1, ledit procédé comportant les étapes suivantes :

-    séparation desdits n composants $C_i$ à l'intérieur de ladite colonne,
-    détection desdits n composants $C_i$ sous la forme dudit premier chromatogramme représentant n pics successifs Pi,

caractérisé en ce que ledit procédé d'identification comporte les étapes suivantes :

-    détermination pour chaque pic Pi de la rétention relative $\alpha_{ij}$ entre ledit pic $P_i$ et un pic différent $P_j$, définie par la relation :

$$\alpha_{ij} = \frac{Tr(i) - Tm}{Tr(j)-Tm},$$

Tr(i) et Tr(j) désignant respectivement les temps de rétention associés aux dits pics $P_i$ et $P_j$, j variant de 0 à i-1 et

$$\alpha_0 = \frac{Tr(0)}{Tm},$$

Tr(0) désignant le temps de rétention du composant $C_0$,
-    identification desdits n composants $C_i$ en déterminant l'appartenance desdites rétentions relatives $\alpha_{ij}$ à des intervalles connus de rétentions relatives.

Ainsi, on calcule pour chaque pic, un jeu de rétentions relatives par rapport aux pics qui le précèdent. Ce jeu de rétentions relatives permet de valider de façon quasi-certaine la présence du composant associé au pic en fonction de l'appartenance des différentes rétentions relatives aux intervalles de rétentions relatives correspondants.

Avantageusement, lesdits intervalles connus de rétentions relatives sont déterminés expérimentalement.

De manière avantageuse, ladite première colonne de chromatographie retient au moins les hydrocarbures suivants : le propane $C_3H_8$, l'iso-butane $iC_4H_{10}$, le normal-butane $nC_4H_{10}$, le néo-pentane $néoC_5H_{12}$, l'iso-pentane $iC_5H_{12}$, le normal-pentane $nC_5H_{12}$.

Avantageusement, tous les composants supérieurs au normal-pentane $nC_5H_{12}$ sont identifiés comme des hydrocarbures en C6 et supérieurs.

Avantageusement, ledit échantillon de Gaz Naturel comportant n' composants $C'_i$ retenus dans une deuxième colonne de chromatographie, n' étant un entier strictement supérieur à 1 et i variant de 0 à n'-1, ladite deuxième colonne de chromatographie retenant au moins les composants suivants : l'azote $N_2$, le méthane $CH_4$, le dioxyde de carbone $CO_2$, l'éthane $C_2H_6$, le propane $C_3H_8$ et l'eau $H_2O$, ledit procédé comportant les étapes suivantes :

-    injection d'un deuxième échantillon dudit Gaz Naturel dans ladite deuxième colonne de chromatographie,
-    séparation desdits n' composants $C'_i$ à l'intérieur de ladite colonne,
-    détection desdits n' composants $C'_i$ sous la forme d'un deuxième chromatogramme représentant n' pics successifs $P'i$,
-    identification desdits n' composants $C'_i$ à partir dudit deuxième chromatogramme comportant les étapes suivantes :

    ○    identification du pic du méthane par comparaison de sa surface et de son temps de rétention à des valeurs prédéterminées,
    ○    identification du pic de l'azote par comparaison de sa surface à une valeur prédéterminée et de son temps de rétention avec le temps de rétention dudit pic du méthane.

Ainsi, on utilise une deuxième méthode d'identification particulièrement adaptée à la colonne de chromatographie, notée CA, permettant de séparer l'azote $N_2$, le méthane $CH_4$, le dioxyde de carbone $CO_2$, l'éthane $C_2H_6$, le propane $C_3H_8$ et l'eau $H_2O$. Les chromatogrammes associés à la colonne CA sont très souvent semblables et la reconnaissance peut presque se faire à l'oeil nu. Ainsi, un chromatogramme de Gaz Naturel, sur la colonne CA, présente toujours un pic majoritaire associé au méthane précédé d'un pic plus petit associé à l'azote. Les seules différences sur ce chromatogramme peuvent venir de la taille des pics proportionnelle à la concentration et dans certains cas, de l'absence de composants tels que le dioxyde de carbone et/ou le propane et/ou l'eau. Une telle méthode d'identification permet également de s'affranchir de l'utilisation de fenêtre temporelle d'identification.

Avantageusement, ladite identification desdits n' composants $C'_i$ à partir dudit deuxième chromatogramme comporte en outre les étapes suivantes:

- calcul pour les pics $P'_i$ restant à identifier du coefficient $dd(i)$ défini par la relation :

$$dd(i) = 100 . \frac{Tr(i) - Tr(CH_4)}{Tr(CH_4)}$$

où $Tr(i)$ désigne le temps de rétention du composant i et $Tr(CH_4)$ désigne le temps de rétention du méthane, avec $Tr(i) > Tr(CH4)$,
- identification de la présence dans ledit Gaz Naturel de dioxyde de carbone $CO_2$ et d'éthane $C_2H_6$ lorsque ledit deuxième chromatogramme comporte deux pics présentant chacun un coefficient $dd(i)$ tel que : $15 < dd(i) < 100$.
- identification de l'absence dans ledit Gaz Naturel de dioxyde de carbone $CO_2$ et d'éthane $C_2H_6$ lorsque ledit deuxième chromatogramme ne comporte aucun pic présentant un coefficient $dd(i)$ tel que : $15 < dd(i) < 100$,
- identification de la présence dans ledit Gaz Naturel de dioxyde de carbone $CO_2$ lorsque ledit deuxième chromatogramme comporte un seul pic présentant un coefficient $dd(i)$ tel que : $15 < dd(i) < 100$ et lorsque ledit coefficient $dd(i)$ est inférieur à 50,
- identification de la présence dans ledit Gaz Naturel d'éthane $C_2H_6$ lorsque ledit deuxième chromatogramme comporte un seul pic présentant un coefficient $dd(i)$ tel que : $15 < dd(i) < 100$ et lorsque ledit coefficient $dd(i)$ est supérieur à 50.

Avantageusement, ladite identification desdits n' composants $C'_i$ à partir dudit deuxième chromatogramme comporte en outre les étapes suivantes :

- calcul pour les pics $P'_i$ restant à identifier du coefficient $dd(i)$ défini par la relation :

$$dd(i) = 100 . \frac{Tr(i) - Tr(CH_4)}{Tr(CH_4)}$$

où $Tr(i)$ désigne le temps de rétention du composant i et $Tr(CH_4)$ désigne le temps de rétention du méthane, avec $Tr(i) > Tr(CH4)$,
- identification de la présence dans ledit Gaz Naturel de propane $C_3H_8$ lorsque ledit deuxième chromatogramme comporte un pic présentant un coefficient $dd(i)$ tel que : $250 < dd(i) < 570$.

Avantageusement, ladite identification desdits n' composants $C'_i$ à partir dudit deuxième chromatogramme comporte en outre les étapes suivantes:

- calcul pour les pics $P'_i$ restant à identifier du coefficient $dd(i)$ défini par la relation :

$$dd(i) = 100 . \frac{Tr(i) - Tr(CH_4)}{Tr(CH_4)}$$

où $Tr(i)$ désigne le temps de rétention du composant i et $Tr(CH_4)$ désigne le temps de rétention du méthane, avec $Tr(i) > Tr(CH4)$,
identification de la présence dans ledit Gaz Naturel d'eau $H_2O$ lorsque ledit deuxième chromatogramme comporte un pic présentant un coefficient $dd(i)$ tel que : $570 < dd(i) < 2000$.

Avantageusement, le procédé selon l'invention comporte les étapes suivantes :

- détermination du pourcentage $\%(C_3H_8)$ de propane dans ladite première colonne,
- détermination du pourcentage $\%'(C_3H_8)$ de propane dans ladite deuxième colonne,

- calcul d'un facteur dit d'alignement entre lesdites première et deuxième colonnes :

$$\frac{\%'(C_3H_8)}{\%(C_3H_8)},$$

- application dudit facteur d'alignement aux pourcentages de tous les composants $C_i$ retenus dans ladite première colonne.

Ainsi, l'application de ce facteur d'alignement permet l'alignement des résultats de la colonne CB sur ceux de la colonne CA.

Avantageusement, ledit propane retenu par ladite première colonne comporte un certain pourcentage d'humidité et ladite détermination du pourcentage $\%(C_3H_8)$ de propane dans ladite première colonne est réalisée en tenant compte du pourcentage d'humidité associé au pic de propane $C_3H_8$ du chromatogramme de ladite première colonne.

Ainsi, le pic associé au propane de la première colonne, telle que la colonne CB, comporte à la fois du propane et de l'humidité présente dans le milieu analytique (échantillon, instrument, lignes de transfert). Comme l'eau est mesurée sur la deuxième colonne CA, il est facile de reconstituer la teneur en propane « sec », c'est à dire sans humidité, de la première colonne avant l'alignement des résultats.

D'autres caractéristiques et avantages de la présente invention apparaîtront dans la description suivante d'un mode de réalisation de l'invention, donné à titre illustratif et nullement limitatif.

Dans les figures suivantes :

- La figure 1 représente un chromatogramme illustrant l'état de la technique,
- La figure 2 représente un premier chromatogramme tel qu'utilisé par le procédé d'identification selon l'invention,
- La figure 3 représente un agrandissement du premier chromatogramme représenté en figure 2,
- La figure 4 représente un deuxième chromatogramme tel qu'utilisé par le procédé d'identification selon l'invention,
- La figure 5 représente un agrandissement du deuxième chromatogramme représenté en figure 4.

La figure 1 a déjà été partiellement décrite en relation avec l'état de la technique. La figure 1 représente également les temps de rétention réduits T'rC et T'rD associés respectivement aux pics C et D et correspondant à la différence entre le temps de rétention et le temps mort. De manière générale, le temps de rétention réduit est donné par la relation : T'r = Tr - Tm.

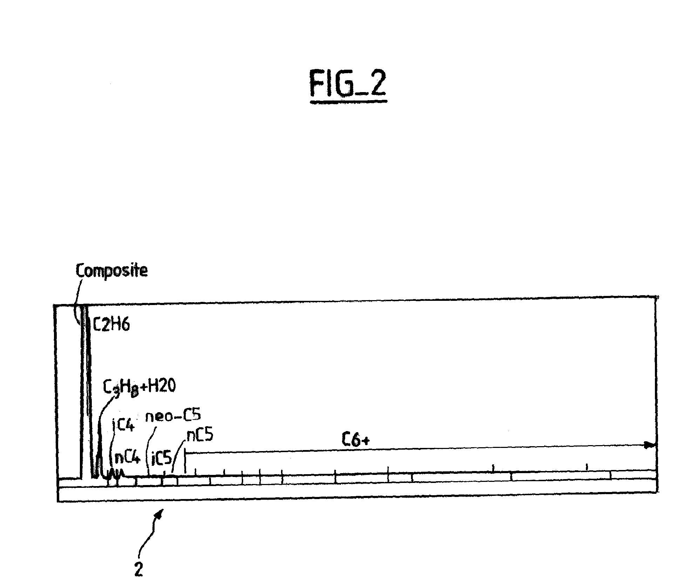

La figure 2 représente un exemple de chromatogramme 2 tel qu'utilisé dans le procédé d'identification selon l'invention. Ce chromatogramme 2 comprend une série de pics associés à la colonne, notée précédemment CB, permettant de séparer une première série de composants tels que le propane $C_3H_8$, l'iso-butane $iC_4H_{10}$, noté iC4, le normal-butane $nC_4H_{10}$, noté nC4, le néo-pentane $néoC_5H_{12}$, noté néo-C5, l'iso-pentane $iC_5H_{12}$, noté iC5, le normal-pentane $nC_5H_{12}$, noté nC5, les hexanes et les composants supérieurs en $C_7$, $C_8$ et $C_9$ notés C6+.

La colonne CB se comporte comme une colonne à distiller et sépare les composants suivant leurs points d'ébullition.

Le chromatogramme 2 comporte un premier massif, noté pic composite, incluant des composants non retenus tels que l'hélium, l'hydrogène, l'argon, l'oxygène, l'azote, le monoxyde de carbone, le méthane et le dioxyde de carbone.

Ce pic composite peut être immédiatement suivi d'un pic mal séparé, l'éthane ($C_2H_6$), non mesuré avec cette colonne.

Le pic de $C_2H_6$ est suivi d'un pic moins important qui correspond au propane $C_3H_8$ et à l'eau $H_2O$ qui ne sont pas séparés. Cette eau représente l'humidité présente dans le milieu analytique (échantillon, instrument, lignes de transfert). L'ensemble comprenant le propane $C_3H_8$ et à l'eau $H_2O$ sera noté C3b par la suite.

Ce pic de propane $C_3H_8$ et d'eau précède une paire correspondant à l'iso-butane iC4 pour le premier pic et au normal-butane nC4 pour le second.

Cette paire précède une autre paire comprenant l'iso-pentane iC5 pour le premier pic et le normal-pentane nC5 pour le second pic.

Un petit pic peut apparaître juste après le normal-butane : il s'agit du néo-pentane néo-C5. Ce pic n'est pas présent sur le chromatogramme 2 mais l'emplacement de ce pic est indiqué.

Tous les composants supérieurs au normal-pentane nC5 sont sommés et exploités comme des hydrocarbures en C6 et supérieurs, notés C6+.

Le procédé selon l'invention permet d'identifier les composants associés aux différents pics du chromatogramme 2.

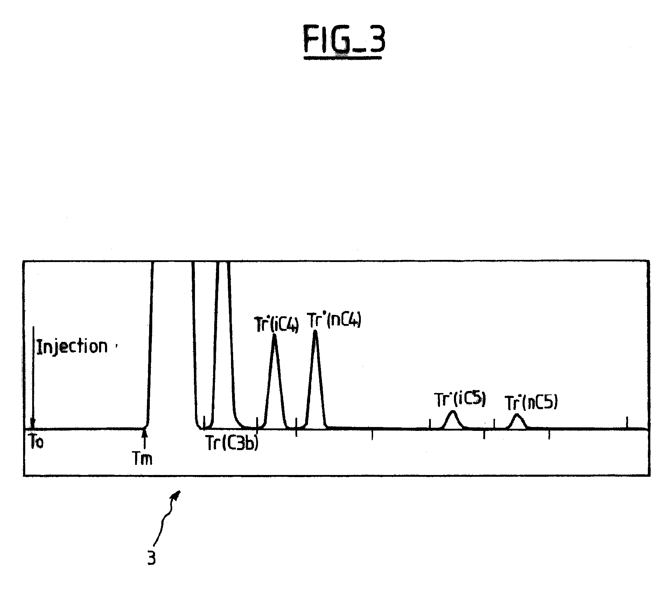

La figure 3 représente un agrandissement 3 du chromatogramme tel que représenté en figure 2 permettant d'illustrer la méthode d'identification selon l'invention.

L'agrandissement 3 représente un temps T0 correspond au temps de départ, de l'injection de l'échantillon de gaz à analyser.

L'agrandissement 3 représente également le pied du pic composite; nous assimilerons le temps de démarrage de ce pic composite au temps mort Tm de la colonne. Ce temps mort Tm correspond à une première déviation de la ligne de base (déterminée par la dérivée seconde du signal), déviation qui précède immédiatement une saturation du signal ou un niveau de signal supérieur à une valeur prédéterminée (ici $10^5$ unités de signal).

La détermination du temps mort Tm permet ensuite de déterminer les temps de rétention réduits T'r(iC4), T'r (nC4), T'r(iC5) et T'r(nC5) respectivement associés aux pics iC4, nC4, iC5 et nC5.

Ces temps de rétention réduits permettent de déterminer la rétention relative $\alpha_{dk}$ entre un pic $P_d$ et un pic $P_k$ précédant le pic $P_d$ définie par la relation :

$$\alpha_{dk} = \frac{Tr(d)\ Tm}{Tr(k) - Tm} = \frac{T'r(d)}{T'r(k)},$$

Tr(d) et Tr(k) désignant respectivement les temps de rétention associés aux pics $P_d$ et $P_k$ et T'r(d) et T'r(k) désignant respectivement les temps de rétention réduits associés aux pics $P_d$ et $P_k$.

On obtient ainsi un jeu de rétentions relatives données dans le tableau 1 et correspondant à l'ensemble des rétentions relatives de chacun des pics par rapport aux pics qui les précèdent ; notons que la rétention relative $\alpha_0$ , définie pour le pic correspondant au premier composant retenu qui est le propane, est donnée par la relation :

$$\alpha_0 = \frac{Tr(C3b)}{Tm}.$$

Tableau 1

| Pic 1 / Pic composite | $\alpha_0 = \dfrac{Tr(C3b)}{Tm}$ |
|---|---|
| Pic 2 / Pic 1 | $\alpha_1 = \dfrac{T'r(iC4)}{T'r(C3b)}$ |
| Pic 3 / Pic 1 | $\alpha_2 = \dfrac{T'r(nC4)}{T''r(C3b)}$ |
| Pic 3 / Pic 2 | $\alpha_3 = \dfrac{T'r(nC4)}{T'r(iC4)}$ |
| Pic 4 / Pic 1 | $\alpha_4 = \dfrac{T'r(iC5)}{T'r(C3b)}$ |
| Pic 4 / Pic 2 | $\alpha_5 = \dfrac{T'r(iC5)}{T'r(iC4)}$ |
| Pic4/Pic3 | $\alpha_6 = \dfrac{T'r(iC5)}{T'r(nC4)}$ |
| Pic 5 / Pic 1 | $\alpha_7 = \dfrac{T'r(nC5)}{T'r(C3b)}$ |
| Pic 5 / Pic 2 | $\alpha_8 = \dfrac{T'r(nC5)}{T'r(iC4)}$ |
| Pic 5 / Pic 3 | $\alpha_9 = \dfrac{T'r(nC5)}{T'r(nC4)}$ |
| Pic5/Pic4 | $\alpha_{10} = \dfrac{T'r(nC5)}{T'r(iC5)}$ |

Chacune des rétentions relatives du tableau 1 est ensuite comparée à des intervalles de rétentions relatives déterminés expérimentalement en faisant varier la température et le débit du gaz vecteur ; chaque intervalle d'identification définit un couple de composants. Ainsi, l'appartenance d'une rétention à un certain intervalle permet d'identifier deux composants. A part le premier pic, tous les composants sont définis par au moins deux rétentions relatives, le nombre de rétentions relatives associé à un composant augmentant avec son temps de rétention. Une seule rétention relative suffit à identifier un composant. Toutefois, les autres rétentions associées à ce même composant permettent de confirmer avec certitude la présence de celui-ci. Ainsi, par exemple, le fait d'avoir un intervalle dans lequel se situe $\alpha_2$ permet de valider la présence de C3b et nC4. La présence de nC4 est ensuite confirmée par la présence de $\alpha_3$ dans un autre intervalle. De même, nC5 peut être identifié par $\alpha_7$ puis confirmé par les rétentions $\alpha_8$, $\alpha_9$ et $\alpha_{10}$.

A titre d'exemple, l'analyse d'un Gaz Naturel a donné les résultats suivants :

| Tm=4.288 | Tr(1)=0.381 | Tr (2)=0.454 | Tr (3)=0.512 |
|---|---|---|---|
| avec Tr(1) comme premier pic mesuré de la colonne CB. | | | |

Les fourchettes de reconnaissances des alphas, haut (h) et bas (b) sont, par exemple, les suivantes :

| $\alpha 0b$ = 1,200 | $\alpha 0h$ = 1.400 | $\alpha 0$ = Tr (C3b) /Tm |
|---|---|---|
| $\alpha 1b$ = 1,500 | $\alpha 1h$ = 1.999 | $\alpha 1$ = T'r (IC4) / T'r (C3b) |

1$^{er}$ pic, recherche du C3 :

$\alpha 0$ = 0.381/0.288 = 1.323
On a : 1.200<1.323<1.400 donc le pic considéré est bien le C3b.

2eme pic, recherche du IC4 :

$\alpha 1$ = (0.454-0.288)/(0.381-0.288) = 1.785
On a : 1.500<1.785<1.999 donc le pic considéré est bien le IC4

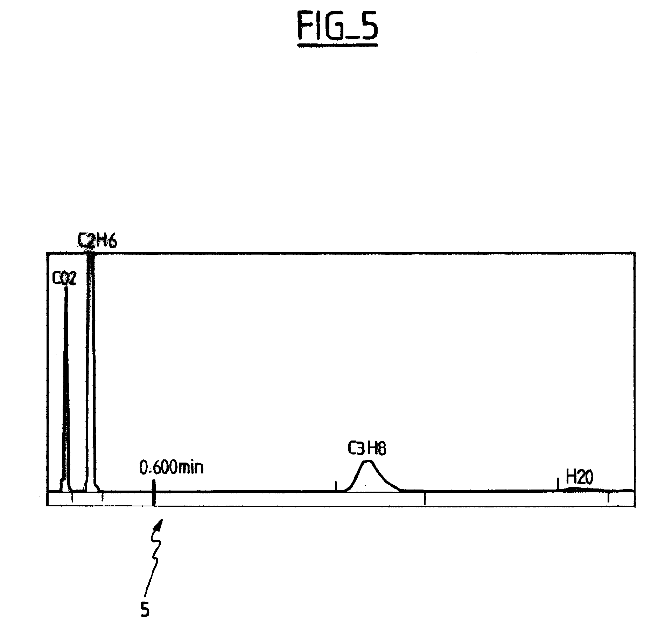

Notons que le premier pic n'est pas nécessairement le C3b. Faisons ainsi l'hypothèse que le premier pic donne une valeur $\alpha 0$ = 1.128 ; cette valeur n'est pas située dans la fourchette et le premier pic n'est donc pas le C3b. Par contre, le deuxième pic donne une valeur de $\alpha 0$ = 1.323 et c'est donc ce deuxième pic qui correspond au C3b.
Les validations et confirmations sont effectuées en cascade par un algorithme d'identification qui compare chaque rétention expérimentale aux différents intervalles de rétention.
En présence de tous les composants, toutes les rétentions relatives doivent être validées par un intervalle.
De manière générale, la rétention relative entre deux pics appartenant à des séries homologues d'hydrocarbures tels que ceux de la colonne CB, varie très peu avec la température de la colonne et le débit du gaz vecteur.
Notons que le néo-pentane ne possède pas de pic spécifique associé sur les chromatogrammes représentés en figures 2 et 3. Le néo-pentane est un composant dont le temps de rétention est très proche de celui du normal-butane n-C4. A faible teneur (inférieure à 50 ppm), il est intégré avec le n-butane ; à plus forte teneur et si la colonne analytique le permet, il est traité individuellement. Pour qu'il soit reconnu comme tel, il faut que la relation suivante soit vérifiée : Tr(nC4)<Tr(néo-C5)<1,1.Tr(nC4).
Notons également que l'absence éventuelle de C3b sur le chromatogramme ne gêne en aucune façon le procédé d'identification puisque le calcul des différentes rétentions relatives à partir du temps mort puis la comparaison de ces rétentions relatives par rapport aux fenêtres de rétentions relatives suffisent à identifier les composants présents.

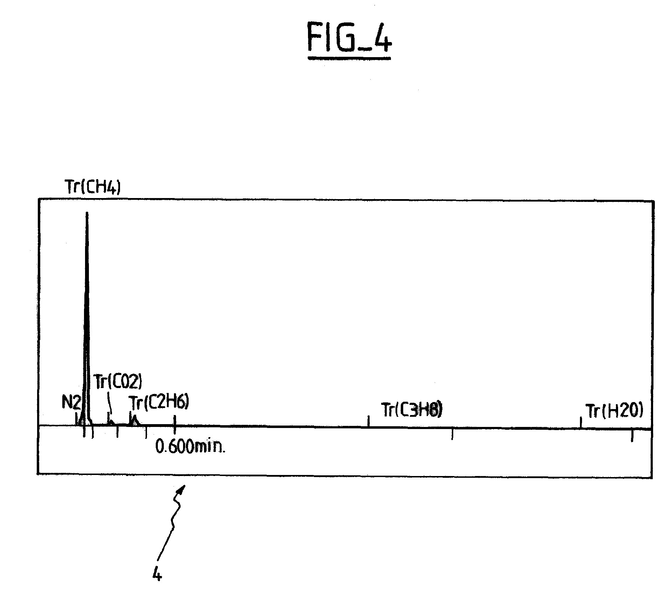

La figure 4 représente un exemple de chromatogramme 4 tel qu'utilisé dans le procédé d'identification selon l'invention. Ce chromatogramme 4 comprend une série de pics associés à la colonne, notée précédemment CA, permettant de séparer une deuxième série de composants plus légers que ceux de la colonne CB tels que l'azote $N_2$, le méthane $CH_4$, le dioxyde de carbone $CO_2$, l'éthane $C_2H_6$, le propane $C_3H_8$ et l'eau $H_2O$.
Le chromatogramme 4 présente un pic majoritaire de méthane $CH_4$ qui est précédé d'un pic plus petit d'azote $N_2$.
Deux pics suivent le pic de méthane et correspondent au dioxyde de carbone $CO_2$ et à l'éthane $C_2H_6$.
Ces deux derniers pics sont généralement suivis de deux autres composants : le propane $C_3H_8$, qui peut se situer entre le tiers et les deux tiers de l'analyse, et l'eau $H_2O$, qui se situe généralement dans le dernier tiers de l'analyse. Les deux pics du propane et de l'eau sont visibles en figure 5 qui représente un agrandissement 5 du chromatogramme 4 de la figure 5.
Le chromatogramme 4 sera sensiblement semblable dans la majorité des cas. Les seules différences pourront venir de la taille des pics qui seront proportionnels aux concentrations et dans certains cas de l'absence de certains composants comme le dioxyde de carbone et/ou le propane et/ou l'eau.
Le procédé selon l'invention permet de déterminer de façon systématique les différents composants associés aux pics du chromatogramme 4.

Identification du Méthane

S'agissant d'un Gaz Naturel, le pic de méthane doit toujours être présent et sa concentration pourra être comprise entre 65 et 99.8%. En dehors de cette plage de concentration, le gaz sera déclaré non conforme. Le méthane est reconnu comme tel lorsqu'un pic ayant un temps de rétention $Tr(CH_4)$ compris entre 0.15 et 0.6 minute présente une surface supérieure à $10^6$ unités de surface.

## Identification de l'Azote

La présence d'une surface supérieure à 200 unités de surface avec un temps de rétention $Ir(N_2)$ qui ne soit pas inférieur de plus de 1.5 secondes à celui du méthane identifie l'Azote.

## Identification du dioxyde de carbone $CO_2$ et de l'éthane $C_2H_6$.

On calcule le coefficient dd(i) défini par la relation :

$$dd(i) = 100 . \frac{Tr(i) - Tr(CH_4)}{Tr(CH_4)}$$

où Tr(i) désigne le temps de rétention du composant i et $Tr(CH_4)$ désigne le temps de rétention du méthane.

Le composant i peut être le dioxyde de carbone CO2, l'éthane $C_2H_6$, le propane $C_3H_8$ ou l'eau $H_2O$.

Le dioxyde de carbone $CO_2$ et l'éthane $C_2H_6$ se situent dans une première zone tel que : 15<dd(i)<100.

Lorsque deux pics sont identifiés dans cette zone, ils correspondent donc respectivement au CO2 et à l'éthane.

Lorsqu'il n'y a pas de pic dans cette zone, il n'y a ni $CO_2$ ni éthane.

Lorsqu'il y a un seul pic dans cette zone et si le coefficient dd(i) associé à ce pic est tel que : dd(i)<50 il s'agira du $CO_2$.

Lorsqu'il y a un seul pic dans cette zone et si le coefficient dd(i) associé à ce pic est tel que : dd(i)>50, il s'agira de l'éthane.

## Identification du propane $C_3H_8$

Le pic du propane $C_3H_8$ est identifié lorsque le chromatogramme 5 comporte un pic présentant un coefficient dd(i) tel que : 250<dd(i)<570.

## Identification de l'eau $H_2O$

Le pic de l'eau $H_2O$ est identifié lorsque le chromatogramme 5 comporte un pic présentant un coefficient dd(i) tel que : 570<dd(i)<2000.

Les valeurs des coefficients dd(i) donnés ci-dessus peuvent être considérées comme très peu dépendantes de la température de la colonne CA et du débit de gaz vecteur. Dès lors, l'identification des composants retenus dans la colonne CA est plus sure qu'une identification utilisant des fenêtres temporelles d'identification.

Une fois l'analyse qualitative (identification des composants du Gaz Naturel) effectuée, aussi bien sur la colonne CA que sur la colonne CB, il est également possible de réaliser une analyse quantitative, c'est à dire d'avoir accès aux concentrations des composants.

Ainsi, la concentration d'un composant est égale au rapport de la surface du pic qui lui est associé sur un coefficient de réponse Kr déterminé pour chaque composant par étalonnage externe.

Le procédé selon l'invention permet d'utiliser avantageusement le propane détecté à la fois sur la colonne CB et sur la colonne CA pour aligner les résultats de la colonne CB sur ceux de la colonne CA via les étapes suivantes :

-   détermination du pourcentage $\%(C_3H_8)$ de propane dans la colonne CB,
-   détermination du pourcentage $\%'(C_3H_8)$ de propane dans la colonne CA,
-   calcul d'un facteur F dit d'alignement entre colonnes CA et CB tel que:

$$F = \frac{\%'(C_3H_8)}{\%(C_3H_8)},$$

-   application du facteur d'alignement aux pourcentages de tous les composants retenus dans la colonne CB.

Le propane C3b de la colonne CB n'est cependant pas pur puisqu'il comporte du propane et de l'humidité présente dans le milieu analytique. Comme l'eau est mesurée dans la colonne CA, il est aisé de reconstituer la teneur de propane « sec » (c'est à dire sans humidité) dans la colonne CB avant l'alignement des résultats entre les deux colonnes.

Pour effectuer l'alignement une double condition est requise: la teneur en propane de la colonne CA doit être supérieure à 600 ppm, et être supérieure ou égale à 1.5 fois la teneur brute en eau, exprimée en équivalent propane.

Pour des raisons de commodité, l'eau est quantifiée sur la colonne CA avec le même facteur de réponse que le propane.

Lors de l'étalonnage le coefficient de réponse du C3b est déterminé comme suit :

$$Kr(C3b) = \frac{\text{concentration\_propane} + \text{concentration\_eau}}{\text{SurfaceC3b}}$$

L'eau n'ayant pas les mêmes facteurs de réponse sur les colonnes CA et CB, il convient de déterminer un facteur de correction, w, que l'on détermine une fois pour toute pour chaque appareil.

Ce facteur w est déterminé comme suit :

a) au moment de l'étalonnage avec un Gaz Naturel contenant plus de 1% de Propane, w est fixé arbitrairement comme étant égal à 0.5.

b) après étalonnage on passe un Gaz Naturel de composition connue contenant quelques centaines de ppm de propane. On ajuste w de façon à retrouver les justes valeurs du propane et autres hydrocarbures de ce dernier Gaz Naturel.

c) on pratique un nouvel étalonnage avec le facteur w fraîchement actualisé.

d) ce facteur sera conservé comme définitif par la suite.

Lors de la quantification, la teneur en eau est équivalente au produit: % eau corrigé = w x concentration eau (en équivalent propane)

Si la teneur en propane est supérieure à 600 ppm et si cette teneur est supérieure à 1.5 fois la concentration en eau exprimée en équivalent propane alors le facteur d'alignement est déterminé par la relation suivante:

$$F = \frac{\%\text{propane\_colonne\_CA}}{\%\text{C3b} - \%\text{eau\_corrigé}}$$

Ce facteur F est alors appliqué à tous les composants de la colonne CB. Tous les composants (colonnes CA et CB) sauf le C3b et l'eau de la colonne CA sont par la suite normalisés à 100%.

La quantification est exprimée en gaz sec. La teneur en eau n'est pas exploitée en tant que mesure car cette humidité peut provenir aussi bien de l'échantillon que des lignes de transfert (effets de surface: adsorption/désorption).

Bien entendu, l'invention n'est pas limitée au mode de réalisation qui vient d'être décrit.

Notamment, le procédé d'identification a été décrit avec deux colonnes différentes CA et CB, le Gaz Naturel étant par exemple injecté via un tube en Y à la fois dans la colonne CA et dans la colonne CB ; toutefois, les procédés d'identification sur les deux colonnes CA et CB peuvent être mis en oeuvre indépendamment l'un de l'autre.

Enfin, on pourra remplacer tout moyen par un moyen équivalent sans sortir du cadre de l'invention.

**Revendications**

1. Procédé d'identification par chromatographie en phase gazeuse d'au moins deux composants $C_d$ et $C_k$ appartenant à un Gaz Naturel, lesdits deux composants $C_d$ et $C_k$ étant retenus dans une première colonne de chromatographie, ledit procédé comportant les étapes suivantes :

- injection d'un premier échantillon dudit Gaz Naturel dans ladite première colonne de chromatographie,
- séparation desdits composants $C_d$ et $C_k$ à l'intérieur de ladite première colonne,
- détection desdits composants $C_d$ et $C_k$ sous la forme d'un premier chromatogramme représentant au moins deux pics successifs $P_d$ et $P_k$,

**caractérisé en ce que** ledit procédé d'identification comporte les étapes suivantes :

- détermination du temps mort Tm à partir dudit premier chromatogramme,
- détermination de la rétention relative $\alpha_{dk}$ entre lesdits pics $P_d$ et $P_k$ définie par la relation :

$$\alpha_{dk} = \frac{Tr(d) - Tm}{Tr(k) - Tm}$$

, Tr(d) et Tr(k) désignant respectivement les temps de rétention associés aux dits pics $P_d$ et $P_k$,
- identification des composants $C_d$ et $C_k$ en déterminant l'appartenance de ladite rétention relative $\alpha_{dk}$ à un intervalle connu de rétentions relatives.

2. Procédé d'identification selon la revendication précédente **caractérisé en ce que** ledit premier chromatogramme comporte un premier pic dit composite, correspondant à un composant non retenu, ledit temps mort Tm étant assimilé au temps de démarrage dudit pic composite.

3. Procédé d'identification selon l'une des revendications précédentes, ledit échantillon de Gaz Naturel comportant n composants $C_i$ retenus dans ladite première colonne de chromatographie, n étant un entier strictement supérieur à 1 et i variant de 0 à n-1, ledit procédé comportant les étapes suivantes :

- séparation desdits n composants $C_i$ à l'intérieur de ladite colonne,
- détection desdits n composants $C_i$ sous la forme dudit premier chromatogramme représentant n pics successifs Pi,

**caractérisé en ce que** ledit procédé d'identification comporte les étapes suivantes :

- détermination pour chaque pic Pi de la rétention relative $\alpha_{ij}$ entre ledit pic $P_i$ et un pic différent $P_j$, définie par la relation :

$$\alpha_{ij} = \frac{Tr(i) - Tm}{Tr(j) - Tm}.$$

Tr(i) et Tr(j) désignant respectivement les temps de rétention associés aux dits pics $P_i$ et $P_j$, j variant de 0 à i-1 et

$$\alpha_0 = \frac{Tr(0)}{Tm}$$

, Tr(0) désignant le temps de rétention du composant $C_0$,
- identification desdits n composants $C_i$ en déterminant l'appartenance desdites rétentions relatives $\alpha_{ij}$ à des intervalles connus de rétentions relatives.

4. Procédé selon la revendication précédente **caractérisé en ce que** lesdits intervalles connus de rétentions relatives sont déterminés expérimentalement.

5. Procédé selon l'une des revendications 3 ou 4 **caractérisé en ce que** ladite première colonne de chromatographie retient au moins les hydrocarbures suivants : le propane $C_3H_8$, l'iso-butane $iC_4H_{10}$, le normal-butane $nC_4H_{10}$, le néo-pentane $néoC_5H_{12}$, l'iso-pentane $iC_5H_{12}$, le normal-pentane $nC_5H_{12}$.

6. Procédé selon la revendication précédente **caractérisé en ce que** tous les composants supérieurs au normal-pentane $nC_5H_{12}$ sont identifiés comme des hydrocarbures en C6 et supérieurs.

7. Procédé selon l'une des revendications 5 ou 6, ledit échantillon de Gaz Naturel comportant n' composants $C'_i$ retenus dans une deuxième colonne de chromatographie, n' étant un entier strictement supérieur à 1 et i variant de 0 à n'-1, ladite deuxième colonne de chromatographie retenant au moins les composants suivants : l'azote $N_2$, le méthane $CH_4$, le dioxyde de carbone $CO_2$, l'éthane $C_2H_6$, le propane $C_3H_8$ et l'eau $H_2O$, ledit procédé comportant les étapes suivantes :

- injection d'un deuxième échantillon dudit Gaz Naturel dans ladite deuxième colonne de chromatographie,

- séparation desdits n' composants C'$_i$ à l'intérieur de ladite colonne,
- détection desdits n' composants C'$_i$ sous la forme d'un deuxième chromatogramme représentant n' pics successifs P'i,
- identification desdits n' composants C'$_i$ à partir dudit deuxième chromatogramme comportant les étapes suivantes :

  ○ identification du pic du méthane par comparaison de sa surface de son temps de rétention à des valeurs prédéterminées,
  ○ identification du pic de l'azote par comparaison de sa surface à une valeur prédéterminée et de son temps de rétention avec le temps de rétention dudit pic du méthane.

8. Procédé selon la revendication précédente **caractérisé en ce que** ladite identification desdits n' composants C'$_i$ à partir dudit deuxième chromatogramme comporte en outre les étapes suivantes :

- calcul pour les pics P'i restant à identifier du coefficient dd(i) défini par la relation :

$$dd(i) = 100 . \frac{Tr(i) - Tr(CH_4)}{Tr(CH_4)}$$

où Tr(i) désigne le temps de rétention du composant i et Tr($CH_4$) désigne le temps de rétention du méthane, avec Tr(i)>Tr(CH4),
- identification de la présence dans ledit Gaz Naturel de dioxyde de carbone $CO_2$ et d'éthane $C_2H_6$ lorsque ledit deuxième chromatogramme comporte deux pics présentant chacun un coefficient dd(i) tel que : 15<dd(i)<100.
- identification de l'absence dans ledit Gaz Naturel de dioxyde de carbone $CO_2$ et d'éthane $C_2H_6$ lorsque ledit deuxième chromatogramme ne comporte aucun pic présentant un coefficient dd(i) tel que : 15<dd(i)<100,
- identification de la présence dans ledit Gaz Naturel de dioxyde de carbone $CO_2$ lorsque ledit deuxième chromatogramme comporte un seul pic présentant un coefficient dd(i) tel que : 15<dd(i)<100 et lorsque ledit coefficient dd(i) est inférieur à 50,
- identification de la présence dans ledit Gaz Naturel d'éthane $C_2H_6$ lorsque ledit deuxième chromatogramme comporte un seul pic présentant un coefficient dd(i) tel que : 15<dd(i)<100 et lorsque ledit coefficient dd(i) est supérieur à 50.

9. Procédé selon l'une des revendications 7 ou 8 **caractérisé en ce que** ladite identification desdits n' composants C'$_i$ à partir dudit deuxième chromatogramme comporte en outre les étapes suivantes :

- calcul pour les pics P'i restant à identifier du coefficient dd(i) défini par la relation :

$$dd(i) = 100 . \frac{Tr(i)-Tr(CH_4)}{Tr(CH_4)}$$

où Tr(i) désigne le temps de rétention du composant i et Tr($CH_4$) désigne le temps de rétention du méthane, avec Tr(i)>Tr(CH4),
- identification de la présence dans ledit Gaz Naturel de propane $C_3H_8$ lorsque ledit deuxième chromatogramme comporte un pic présentant un coefficient dd(i) tel que : 250<dd(i)<570.

10. Procédé selon la revendication précédente **caractérisé en ce qu'**il comporte les étapes suivantes :

- détermination du pourcentage %($C_3H_8$) de propane dans ladite première colonne,
- détermination du pourcentage %'($C_3H_8$) de propane dans ladite deuxième colonne,
- calcul d'un facteur dit d'alignement entre lesdites première et deuxième colonnes :

$$\frac{\%'(C_3H_8)}{\%(C_3H_8)},$$

- application dudit facteur d'alignement aux pourcentages de tous les composants C$_i$ retenus dans ladite pre-

mière colonne.

**11.** Procédé selon la revendication précédente **caractérisé en ce que** ledit propane retenu par ladite première colonne comporte un certain pourcentage d'humidité, ladite détermination du pourcentage %($C_3H_8$) de propane dans ladite première colonne étant réalisée en tenant compte du pourcentage d'humidité associé au pic de propane $C_3H_8$ du chromatogramme de ladite première colonne.

**12.** Procédé selon l'une des revendications 7 à 11 **caractérisé en ce que** ladite identification desdits n' composants $C'_i$ à partir dudit deuxième chromatogramme comporte en outre les étapes suivantes :

- calcul pour les pics $P'i$ restant à identifier du coefficient dd(i) défini par la relation :

$$dd(i) = 100. \frac{Tr(i) - Tr(CH_4)}{Tr(CH_4)}$$

où Tr(i) désigne le temps de rétention du composant i et Tr($CH_4$) désigne le temps de rétention du méthane, avec Tr(i)>Tr(CH4),
- identification de la présence dans ledit Gaz Naturel d'eau $H_2O$ lorsque ledit deuxième chromatogramme comporte un pic présentant un coefficient dd(i) tel que : 570<dd(i)<2000.

FIG_1

# FIG_2

# FIG_3

# FIG_4

# FIG_5

**Office européen des brevets**

**RAPPORT DE RECHERCHE EUROPEENNE**

Numéro de la demande

EP 04 30 0637

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | EP 1 061 365 A (ARVI S N C) 20 décembre 2000 (2000-12-20) * abrégé * * alinéas [0016] - [0031] * * figures 1,2A,2B,3A,3B * ----- | 1-7 | G01N30/46 G01N30/86 |
| Y | GB 982 497 A (ERNST PALM) 3 février 1965 (1965-02-03) * page 1, ligne 12 - ligne 55 * ----- | 1-7 | |
| A | US 2003/015019 A1 (O'BRIEN ROBERT) 23 janvier 2003 (2003-01-23) * abrégé * * alinéas [0003] - [0008] * * alinéa [0041] * * alinéa [0160] * * alinéa [0200] * * alinéa [0363] * * alinéa [0378] * * tableau 1 * ----- | 7-12 | |
| A | WO 03/001196 A (BEZZOLA CARLO ;GEOLOG S P A (IT)) 3 janvier 2003 (2003-01-03) * abrégé * * page 1, ligne 8-28 * * page 3, ligne 1-10 * * page 10, ligne 8 - ligne 15 * * page 10, ligne 23 - page 11, ligne 5 * * page 14, ligne 1 - ligne 10 * ----- | 1-7 | **DOMAINES TECHNIQUES RECHERCHES** (Int.Cl.7) G01N |
| A | FR 2 230 405 A (HALASZ ISTVAN) 20 décembre 1974 (1974-12-20) * page 12, ligne 17 - ligne 37 * * tableau 1 * ----- | 1,8,9,12 | |

Le présent rapport a été établi pour toutes les revendications

| Lieu de la recherche | Date d'achèvement de la recherche | Examinateur |
|---|---|---|
| Munich | 28 janvier 2005 | Bravin, M |

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 04 30 0637

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-01-2005

| Document brevet cité au rapport de recherche | | Date de publication | Membre(s) de la famille de brevet(s) | | Date de publication |
|---|---|---|---|---|---|
| EP 1061365 | A | 20-12-2000 | IT | MI991349 A1 | 18-12-2000 |
| | | | EP | 1061365 A1 | 20-12-2000 |
| GB 982497 | A | 03-02-1965 | DE | 1298313 B | 26-06-1969 |
| US 2003015019 | A1 | 23-01-2003 | US | 2004035183 A1 | 26-02-2004 |
| | | | AU | 2977301 A | 07-08-2001 |
| | | | CA | 2431615 A1 | 02-08-2001 |
| | | | EP | 1257813 A1 | 20-11-2002 |
| | | | JP | 2003521688 T | 15-07-2003 |
| | | | WO | 0155714 A1 | 02-08-2001 |
| WO 03001196 | A | 03-01-2003 | IT | MI20011329 A1 | 23-12-2002 |
| | | | CA | 2451367 A1 | 03-01-2003 |
| | | | WO | 03001196 A1 | 03-01-2003 |
| | | | EP | 1399736 A1 | 24-03-2004 |
| | | | US | 2004234414 A1 | 25-11-2004 |
| FR 2230405 | A | 20-12-1974 | DE | 2313073 A1 | 26-09-1974 |
| | | | BE | 812425 A1 | 01-07-1974 |
| | | | CH | 590688 A5 | 15-08-1977 |
| | | | DD | 110248 A5 | 12-12-1974 |
| | | | FR | 2230405 A1 | 20-12-1974 |
| | | | GB | 1460315 A | 06-01-1977 |
| | | | IL | 44300 A | 31-08-1977 |
| | | | IT | 1011548 B | 10-02-1977 |
| | | | JP | 1031251 C | 29-01-1981 |
| | | | JP | 50007777 A | 27-01-1975 |
| | | | JP | 55023761 B | 25-06-1980 |
| | | | NL | 7403184 A ,B, | 18-09-1974 |
| | | | SU | 660570 A3 | 30-04-1979 |
| | | | US | 3956179 A | 11-05-1976 |
| | | | ZA | 7401658 A | 26-02-1975 |

EPO FORM P0460

Pour tout renseignement concernant cette annexe : voir Journal Officiel de l'Office européen des brevets, No.12/82